**Europäisches Patentamt**

**European Patent Office**

**Office européen des brevets**

(11) Numéro de publication : **0 461 988 B1**

## FASCICULE DE BREVET EUROPEEN

(45) Date de publication du fascicule du brevet :
05.01.94 Bulletin 94/01

(51) Int. Cl.$^5$ : **B01J 8/02,** C07C 29/152

(21) Numéro de dépôt : **91401533.4**

(22) Date de dépôt : **11.06.91**

(54) **Reacteur avec une paroi inferieure et/ou une paroi superieure comportant une couche d'un materiau souple réfractaire et son utilisation.**

(30) Priorité : **15.06.90 FR 9007614**
**15.06.90 FR 9007615**
**16.05.91 FR 9105997**

(43) Date de publication de la demande :
**18.12.91 Bulletin 91/51**

(45) Mention de la délivrance du brevet :
**05.01.94 Bulletin 94/01**

(84) Etats contractants désignés :
**BE DE ES FR GB GR IT NL SE**

(56) Documents cités :
**US-A- 2 961 304**
**US-A- 4 374 095**

(73) Titulaire : **INSTITUT FRANCAIS DU PETROLE**
**4, avenue de Bois Préau**
**F-92502 Rueil-Malmaison (FR)**

(72) Inventeur : **Poussin, Bernard**
**5 Rue de la Forme**
**F-78240 Carrieres sur Seine (FR)**

EP 0 461 988 B1

Jouve, 18, rue Saint-Denis, 75001 PARIS

## Description

La présente invention concerne un réacteur pour effectuer des réactions chimiques en phase gazeuse à une température et à une pression déterminée et son utilisation. Elle concerne aussi un réacteur pour effectuer un procédé de filtration de particules dans une phase liquide.

Ce réacteur opérant en phase gazeuse peut s'appliquer au reformage des coupes pétrolières, à l'aromatisation de coupes pétrolières et à la synthèse du méthanol.

L'invention concerne plus particulièrement les réacteurs d'axe vertical comportant au moins un compartiment rempli d'un lit de particules traversé par un fluide monophasique et de préférence gazeux, pouvant opérer à une pression de 1 à 200 bar par exemple.

L'art antérieur est illustré par le brevet US 2,961,304 dans lequel l'auteur diminue les pertes de charge en filtrant la charge gazeuse ou liquide en fond de réacteur où s'effectue l'alimentation et en évitant une fluidisation du lit par la présence, en tête du réacteur, d'un grillage tricoté sur le lit de particules, sur lequel repose des particules de solides inertes. Ce grillage n'est donc pas étanche et n'est pas souple.

Par ailleurs, l'arrière plan technologique est illustré par le brevet US 4,374,095 qui décrit un pot catalytique dans lequel les gaz d'échappement ne peuvent circuler qu'axialement à travers des éléments catalytiques contenus dans une enveloppe de fibres céramiques.

Enfin, il est connu d'utiliser un réacteur de type radial, de forme cylindrique où le fluide est introduit par une couronne près de la virole dans l'épaisseur d'un garnissage particulaire à base de catalyseur, de forme annulaire. Ce fluide traverse le lit catalytique radialement et l'effluent est collecté dans une cheminée centrale perforée où il est ensuite évacué.

La tête du compartiment de particules est généralement composé d'un chapeau coiffant la cheminée, qui plonge dans le lit catalytique et d'un ensemble comprenant un couvercle et une jupe constitués de secteurs boulonnés et démontables qui pénètrent dans le lit catalytique surmonté d'une couche de billes inertes de granulométrie appropriée. Cette jupe pénètre dans le lit sur une longueur d'environ 0,5 fois l'épaisseur de celui-ci, cette pénétration représentant le minimum qui doit subsister après retassage supposé du lit catalytique après la mise en température et en pression, afin d'obliger le flux gazeux à parcourir au moins une fois l'épaisseur du lit.

La conception de cet ensemble impose des contraintes de montage et d'assemblage; l'ajustage des différents éléments s'avère difficile: il faut en effet couper ou rallonger la jupe en fonction de la quantité de catalyseur à charger, de sa propre densité et de sa densité de remplissage, sans négliger les problèmes liés à la fragilisation de la boulonnerie après utilisation.

Par ailleurs, en tête de lit, la quantité de catalyseur emprisonnée à l'intérieur et à l'extérieur de la jupe travaille peu ou pas du tout, de sorte qu'un volume de catalyseur, généralement cher, pouvant représenter environ 8 à 15 % du volume total du compartiment est inutilisé, ce qui grève de ce fait l'économie du procédé réalisé.

En fond de lit, il en est de même : la hauteur de garde des moyens de collecte sur la base de la cheminée correspondant à la hauteur du lit parcourue par l'effluent gazeux de façon à parcourir au moins une fois l'épaisseur du lit, crée une zone morte qui est préjudiciable à l'économie du procédé.

Un premier objet de l'invention est d'éviter les zones mortes en tête de lit dans le compartiment particulaire. Un autre objet est d'éviter les zones mortes en fond de lit, ces deux objets permettant d'atteindre la capacité maximale de remplissage du compartiment et un optimum de son utilisation.

Un autre objet de l'invention est de pouvoir disposer d'une flexibilité de remplissage du compartiment en fonction de la demande souhaitée avec un minimum de contraintes et donc avec une rapidité accrue.

Un autre objet de l'invention est de pouvoir adapter la couverture du lit au volume du garnissage au cours de la marche du réacteur en température et en pression et de manière générale, quel que soit le type de charge liquide ou gazeuse, de suivre le tassement du lit grâce à une nouvelle couverture souple épousant la forme du lit selon un plan globalement radial.

Un autre objet de l'invention est d'éviter la présence de billes ou de particules reposant sur le lit catalytique, et la séparation des billes et du catalyseur lors du déchargement.

L'invention propose donc une nouvelle couverture de compartiment comportant un garnissage, évitant notamment le passage du gaz selon un trajet globalement axial et favorisant la traversée de l'épaisseur du garnissage selon un plan sensiblement radial.

Plus précisément l'invention concerne un réacteur pour effectuer des réactions chimiques en phase gazeuse, à une température donnée comprenant une entrée (3) d'une charge gazeuse et une sortie (9) d'un effluent, une enveloppe (7) annulaire de distribution de la charge, en communication avec l'entrée de ladite charge, au moins un compartiment (8) annulaire entre ladite enveloppe et ladite sortie comprenant au moins un garnissage (31) sous forme de particules, dans lequel circule radialement ou transversalement au moins un gaz ou un liquide, le compartiment comportant en outre une paroi supérieure (32), caractérisé en ce qu'il comporte, en guise de paroi supérieure, au moins une couche d'au moins un matériau souple (19) de forme appropriée, en matière réfractaire, sensiblement inerte et sensiblement imperméable ou ayant une texture et une porosité telles que ladite couche crée une perte de charge supérieure à celle engendrée par le garnissage de particules, ladite couche de matériau étant disposée sur le garnissage (31) de telle façon qu'elle assure un recouvrement sensiblement total dudit garnissage.

Par matériau souple, on entend un tissu ou un matériau de type feutre ou une superposition de tissu et de matériau de type feutre.

Par matériau de forme appropriée, on entend un matériau souple épousant sensiblement la géométrie de la section du compartiment qui peut être de section carrée ou rectangulaire et avantageusement annulaire.

Evitant la présence de billes ou de matériau particulaire, on maximise le volume disponible pour le catalyseur.

De manière avantageuse, la couche de matériau souple peut coopérer avec une première couche sensiblement inerte de billes (1, 12, 13) ou d'un matériau particulaire de granulométrie et de poids appropriés qui repose sur ladite couche de matériau (19).

Selon un premier mode de mise en oeuvre, ce matériau peut être constitué d'un seul morceau ou d'une pluralité de morceaux cousus et agencés de telle façon qu'ils constituent un seul morceau sensiblement étanche.

Le réacteur comprend généralement au moins un compartiment de catalyseur. Ce compartiment peut être de forme parallélépipédique à section sensiblement rectangulaire ou carrée. Il peut être de forme sensiblement cylindrique à section sensiblement annulaire avec une enveloppe de distribution d'un gaz ou d'un liquide tout autour du compartiment cylindrique et une cheminée centrale sensiblement cylindrique comportant des moyens de collecte d'un effluent. Dans ces cas de figure, la surface de la couche de ce matériau est en général au moins égale à la surface du compartiment selon un plan radial ou transversal occupée par le garnissage traversé par les gaz ou le liquide. La cheminée peut être recouverte par un chapeau métallique ou par un chapeau constitué dudit matériau souple réfractaire sensiblement étanche, la couche du matériau constituant le toit du compartiment et le chapeau étant réalisés d'un seul morceau ou d'une pluralité de morceaux taillés et cousus de façon adéquate pour être sensiblement étanches.

Pour des raisons de commodité on considère le cas où le garnissage est un catalyseur traversé par un gaz ou un mélange de gaz.

Selon un autre mode de réalisation, le compartiment du réacteur peut comporter en outre une paroi inférieure (33) ou plancher comprenant au moins une couche (17) dudit matériau souple de forme appropriée qui coopère éventuellement avec une deuxième couche sensiblement inerte de billes (11, 12, 13) ou d'un matériau particulaire de granulométrie appropriée et qui repose sur ladite deuxième couche inerte de façon à assurer un recouvrement sensiblement total de la deuxième couche.

Selon un deuxième mode de mise en oeuvre, le réacteur comporte en guise de paroi supérieure, au moins une couche comportant une pluralité de bandes ou de pièces d'un tissu en matière réfractaire, sensiblement inerte et sensiblement imperméable ou ayant une texture et une porosité telles que lesdites bandes créent une perte de charge supérieure à celle engendrée par le garnissage de particules, lesdites bandes coopérant éventuellement avec une première couche inerte de billes ou d'un matériau particulaire de granulométrie et de poids appropriés qui repose sur lesdites bandes et étant disposées sur ledit garnissage de telle façon que lesdites bandes assurent un recouvrement sensiblement total dudit garnissage et qu'elles soient au moins en partie superposées de manière à glisser les unes sur les autres dans au moins une direction.

Par bandes de matériau, on entend une coupe pouvant avoir une forme aussi bien sensiblement rectangulaire ou carrée qu'une forme correspondant à un secteur annulaire.

De manière avantageuse, lesdites bandes coopèrent avec la première couche de billes ou de matériau à une meilleure étanchéïté axiale et à une plus grande sécurité d'utilisation, le poids des particules venant s'ajouter au poids du matériau souple et à la pression exercer par la charge à l'entrée du réacteur.

Selon un autre mode de réalisation, le compartiment du réacteur peut comporter en outre une paroi inférieure ou plancher constituée d'au moins une couche comportant une pluralité de bandes dudit matériau, lesdites bandes coopérant éventuellement avec une deuxième couche inerte de billes ou d'un matériau particulaire de granulométrie appropriée et reposant sur ladite deuxième couche inerte selon une disposition telle que lesdites bandes assurent un recouvrement sensiblement total de ladite deuxième couche et telle qu'elles peuvent glisser librement les unes sur les autres dans au moins une direction.

En absence de billes ou de matériau particulaire, la couche souple peut reposer sur le fond du réacteur, qui peut être soit une ou plusieurs plaques métalliques soit un remplissage du fond par du ciment jusqu'à un niveau sensiblement horizontal.

Selon une caractéristique de l'invention, les bandes de matériau souple peuvent déterminer des zones de superposition qui ont une largeur au moins égale à l'amplitude de la déformation que le garnissage peut imposer. Le recouvrement partiel des bandes sous-jacentes favorise de la sorte le glissement libre des bandes et une étanchéité satisfaisante.

Selon une autre caractéristique du réacteur, une partie d'une bande ou d'une pièce dudit matériau peut reposer sur une partie d'une bande contiguë de telle sorte qu'une seule couche constitue la couverture du lit catalytique.

Selon une autre caractéristique, lorsque deux couches de bandes en ce matériau sont superposées, une bande de la couche supérieure peut reposer sur une partie de la largeur de deux bandes contiguës de la couche inférieure, les deux bandes ci-dessus pouvant être jointives ou pas.

Selon une autre caractéristique, lorsque deux bandes dudit matériau sont superposées, une paire d'une bande et une partie d'une autre bande contigüe de la couche supérieure peuvent recouvrir au moins en partie une bande d'une couche inférieure.

Pour avoir les meilleurs résultats il est avantageux de satisfaire au moins une des conditions suivantes :
a) la largeur de la zone de recouvrement des bandes est fonction de la hauteur H du lit catalytique. Elle est habituellement comprise entre 0,001 H et 0,2 H et de préférence comprise entre 0,01 H et 0,1 H.
b) elle peut être aussi fonction d'une dimension ou de l'autre dimension d'une bande. Par exemple la zone de recouvrement peut représenter de 0,05 à 0,5 fois la longueur ou la largeur d'une bande selon que le recouvrement est réalisé selon une direction respectivement radiale ou circulaire et de préférence de 0, 1 à 0,2 fois.
c) la surface de recouvrement peut être par exemple comprise entre 0,05 fois et 1,5 fois la surface du lit et avantageusement comprise entre 0,1 fois et 0,5 fois la surface du lit.

L'étanchéité axiale ainsi réalisée due à la pression des gaz à l'entrée et au poids du matériau particulaire sur la couverture du lit et l'absence de zones mortes dans le lit permettent d'améliorer l'efficacité des zones traversées par une meilleure utilisation du garnissage, en l'occurrence du catalyseur. Par ailleurs, la mise en oeuvre du dispositif est facile dans la mesure ou cet équipement léger ne nécessite pas d'outillage particulier et son temps de mise en place est très court. En effet, il n'y a généralement pas d'ajustage ou de recoupage de la couverture à prévoir en fonction de la hauteur définitive de catalyseur qui est fonction de la quantité souhaitée par le client, la densité propre du catalyseur et la densité du chargement (chargement dense ou chargement à la manche).

La solution proposée avec un toit et éventuellement avec un plancher de compartiment catalytique constitué du matériau souple coopérant avec les couches de billes est particulièrement bien adaptée pour les réacteurs de petite dimension, par exemple pour les réacteurs cylindriques à cheminée centrale, de diamètre par exemple égal à 1.400 mm aussi bien que pour les grands réacteurs.

Ces modes particulièrement avantageux de couverture et de plancher de compartiment annulaire assurent le maintien de l'étanchéité dans la partie haute du réacteur lors du tassement du catalyseur et empêchent l'intrusion du catalyseur dans sa partie basse lors du chargement.

Selon une caractéristique de la couche du matériau souple , celle ci peut être constituée d'au moins un tissu ou d'au moins deux tissus de même nature ou de nature différente, assemblés de préférence par couture.

Selon une autre caractéristique de ladite couche, celle-ci peut être constituée d'au moins un matériau de type feutre ou d'au moins deux matériaux de type feutre de même nature ou de nature différente, assemblés de préférence par couture. Ce matériau peut être plus ou moins pressé de façon à atteindre une épaisseur comprise par exemple entre 0,5 et 50 mm et une densité apparente par exemple de 0,05 à 0,25.

Selon une autre caractéristique de ladite couche, celle-ci peut être constituée d'au moins un tissu et d'au moins un matériau de type feutre, le tissu et ce matériau de type feutre étant avantageusement assemblés par couture.

On a obtenu d'excellents résultats lorsque ladite couche comprend une structure en sandwich, c'est-à-dire au moins un matériau de type feutre disposé entre au moins deux tissus, assemblée avantageusement par couture.

Par exemple si T est le tissu et F le feutre, on peut réaliser un empilement tel que T, F, T ou TFFT, ou TFTFT ou TTFTT ou TT, FF, TT.

Par tissu, on entend un matériau obtenu par l'assemblage de fils entrelacés de même nature ou de nature différente. Par matériau de type feutre, on entend un matériau obtenu par aggrégation intime de flocons et/ou de fils de même nature ou de nature différente.

Lorsque la couverture du compartiment est constituée de bandes superposées, la dimension de celle-ci une fois les bandes mises en places, peut être supérieure à l'épaisseur du garnissage que la charge doit traverser. Dans ce cas, l'extrêmité de chaque bande ou secteur annulaire, côté cheminée par exemple, peut être recouverte par une couronne en ledit matériau souple en matière réfractaire dont la partie supérieure peut être fixée à la cheminée par tout moyen approprié et avec une partie inférieure disposée au dessus de ladite extrêmité de telle façon que les extrémités des bandes ou secteurs annulaires coulissent sous la couronne. Selon une autre variante, l'extrêmité de chaque bande côté cheminée peut recouvrir la partie inférieure de ladite couronne de telle façon qu'il y ait une possibilité de coulissement de ladite extrêmité sur cette couronne.

Le tissu utilisé selon l'invention est constitué de fibres généralement en matière céramique et ayant une bonne résistance à la traction même à haute température par exemple de 15.000 à 28.000 kg/cm² entre 370 et 540°C. Ils ont en général une porosité comprise entre 2 et 8 % et avantageusement entre 3 et 5 %.

- Ils sont souples et résistants aux déformations ;
- Ils résistent à des températures supérieures à 1.200°C ;
- Ils peuvent être combinés avec d'autres oxydes de métaux (alcalins, alcalino-terreux, fer, titane, bore, par exemple) augmentant leur résistance mécanique et/ou leur étanchéité. On utilise, par exemple, le textile ZETEX (marque déposée) dont la composition des fibres est avantageusement la suivante :

Oxyde de silicium    52-60 %
Oxyde de calcium    16-25 %
Oxyde d'aluminium    10-13 %
Oxyde de bore    8-13 %
Oxyde de sodium    0-1 %
Oxyde de Magnesium    0-6 %

et dont la résistance à la traction est par exemple d'environ 17.400 kg/cm² à 540°C.

Selon un autre mode d'utilisation, le tissu peut être rendu sensiblement étanche par dépôt d'une couche l'aluminium-mylar par exemple.

On peut utiliser un tissu tricoté à base de fils en acier réfractaire inoxydable par exemple 304 L ou 316 L de la Société GANTOIS - Saint Dié - France.

On peut aussi utiliser le tissu "KATISS" composé d'une nappe fibre céramique KERLANE 45, résistant jusqu'à plus de 1.260°C, renforcée sur ses deux faces d'un tissu de verre E (sillione) de porosité 2 à 5 %). Il a le plus souvent la composition suivante :

$Al_2O_3$    47 %
$SiO_2$    52 %
$Fe_2O_3 + TiO_2$    $\leq 0,20$ %
$CaO + MgO$    $\leq 0,15$ %
$Na_2O + K_2O$    $\leq 0,25$ %

Le tissu composé de fibres réfractaires CERAFIBER renforcées par des fils d'inconel résistant à 1.260°C en atmosphère oxydante normale peut aussi être utilisé. Il comprend:

$Al_2O_3$    46,5 %
$SiO_2$    53 %
$Fe_2O_3$    0,1 %
$TiO_2$    0,05 %
MgO    0,01 %
CaO    0,04 %
$Na_2 + K_2O$    0,2 %

On a obtenu d'excellents résultats avec les tissus HEXCEL GENIN 1003 et surtout avec les tissus HEXCEL GENIN 1217 provenant de la société HEXCEL GENIN à Décines-Charpieu en France, entre lesquels est mis en sandwich un matériau de type feutre KERLANE 45 (marque déposée)

La composition de ces tissus et feutre est présentée dans le tableau ci-dessous.

| | $SiO_2$ | $Al_2O_3$ | $B_2O_3$ | CaO | MgO | $TiO_2$ | $Na_2O$ | C |
|---|---|---|---|---|---|---|---|---|
| Hexcel Genin 1003 | 83,98 | 0,66 | 0,20 | 0,42 | <0,05 | 0,35 | <0,05 | <0,2 |
| Hexcel Genin 1217 | 24,17 | 62,37 | 15,62 | <0,05 | <0,05 | <0,05 | <0,05 | <0,2 |
| Kerlane 45 | 52 | 47 | | <0,15 | <0,15 | <0,2 | <0,25 | |

A titre d'exemple de feutre métallique, on peut citer le feutre BEKAERT 316 L en acier inoxydable.

Les billes ou matériau particulaire qui reposent en au moins une couche sur la couverture en tissu du lit catalytique ou sur lesquelles repose le plancher en tissu selon l'invention sont en général sensiblement inertes. Elles peuvent être constituées d'un support de catalyseur. Lorsqu'une ou plusieurs couches sont utilisées, la couche en contact avec le matériau souple selon l'invention est en général de faible granulométrie, par exemple 0,5 à 0,8 cm alors que la granulométrie de l'autre couche lorsqu'il y en a deux atteint par exemple 2 à 3 cm. Des billes en alumine ou en mélange alumine-oxyde de silice sont génialement utilisées.

L'invention sera mieux comprise au vu des différentes figures illustrant de manière schématique des modes de réalisation du réacteur, parmi lesquelles :
- la figure 1 montre une coupe longitudinale d'un réacteur radial ;
- les figures 2 et 3 représentent une tête et un fond de lit catalytique avec respectivement un morceau de tissu et une pluralité de morceaux de tissu assemblés par une couture ;
- les figures 4, 5, 6 et 7 illustrent des variantes où l'étanchéité est maintenue le long de la cheminée ou le long du chapeau coiffant la cheminée du réacteur annulaire.
- les figures 8 et 9 représentent une tête et un fond de lit catalytique comprenant une pluralité de bandes superposées de tissu approprié ;
- la figure 10 illustre une variante avec coulissement des tissus, maintenant l'étancheïté le long de la cheminée centrale ;
- la figure 11 montre un exemple de superposition des bandes de tissu.

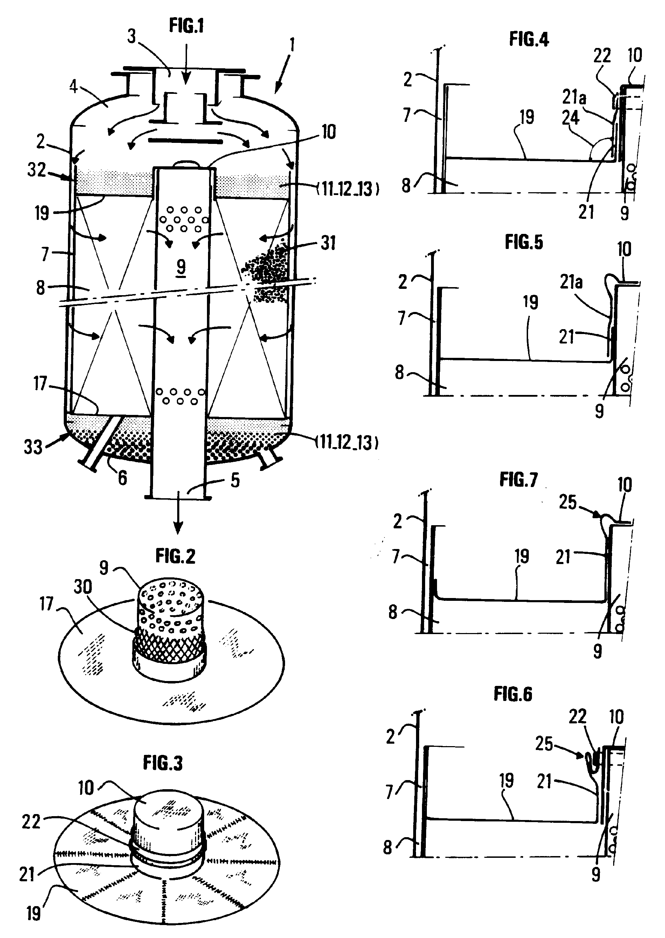

Selon la figure 1, un réacteur cylindrique (1) de type radial adapté à travailler en température et sous pression comprend une virole (2) métallique de forme cylindrique avec une entrée (3) d'une charge gazeuse au niveau du fond supérieur (4) et une sortie (5) d'un effluent au niveau du fond inférieur (6). Tout autour de la virole et calée à l'intérieur du réacteur, une couronne annulaire (7) percée d'ouvertures distribue la charge de manière sensiblement radiale dans un compartiment (8) catalytique de forme annulaire. Le catalyseur est constitué d'un lit comprenant des billes, des pastilles ou plus généralement des bâtonnets de granulométrie égale à environ 1 à 2 mm de diamètre et 4 à 6 mm de long. L'effluent après avoir traversé radialement le lit catalytique, est récupéré par une cheminée (9) centrale de forme cylindrique qui est en général un tube perforé recouvert d'une grille (30) (figure 2) et est évacué par la sortie (5). Au-dessus de la cheminée, un chapeau métallique (10) entourant la partie supérieure de celle-ci, plonge dans le lit catalytique et assure l'étanchéité de la cheminée vis-à-vis de la charge.

Le fond inférieur (6) du réacteur (figure 2) est rempli d'une épaisseur (50 cm) de billes ( 11 ) en céramiques d'alumine ou alumine et oxyde de silice, par exemple de granulométrie environ égale à 1,9 cm, sur laquelle repose une autre épaisseur (12) de 10 cm de billes de nature sensiblement identique, de granulométrie environ égale à 0,63 cm et enfin une troisième épaisseur (13) de 5 cm de support de catalyseur inerte. Sur cette troisième épaisseur est disposée une couche comprenant deux épaisseurs de tissu (17) en fibres de matière céramique Hexcel Genin 1217 (marque déposée), prenant en sandwich une épaisseur d'un matériau type feutre (Kerlane 45) assemblé par couture. Pour des raisons de simplicité, on désignera l'ensemble par tissu, dans la suite de la description . Cette couche sensiblement étanche est constituée d'un seul morceau de forme sensiblement annulaire qui a été taillé à la forme de la surface du compartiment. De préférence, cette couche a été découpée à une surface plus grande qu'il n'en faut pour recouvrir le compartiment de sorte qu'au moins une extrémité, de préférence côté cheminée, peut être redressée contre la paroi de celle-ci. Le catalyseur repose sur le tissu et son poids contribue à l'étanchéité axiale de la paroi inférieure du compartiment.

Le fond (4) supérieur du réacteur comprend au dessus du lit catalytique sur lequel elle s'appuie, une couche dudit matériau selon l'invention (19) en matière céramique décrite ci-dessus. Une première épaisseur (11) de 10 cm de billes ou d'un matériau particulaire de granulométrie par exemple égale à 1,9 cm et une seconde épaisseur (12) de billes de 10 cm en contact avec la couche de tissu, de granulométrie par exemple égale à 0,63 cm reposent sur le tissu. Ces deux couches exercent sur le lit une charge uniformément répartie et contribuent avec la couche de tissu à une étanchéité axiale du lit. Le flux gazeux doit en effet contourner la couverture dès son entrée dans le réacteur pour entrer en contact latéralement avec le lit catalytique, ledit flux circulant radialement ou tranversalement du haut vers le bas, vers la sortie.

La couche souple est constituée d'une pluralité de morceaux dudit matériau ayant chacun une forme de secteur annulaire, mis bout à bout de façon à être cousus de manière connue par un fil indestructible à la chaleur. La couche souple de tissus peut être fixée par des moyens appropriés tels que des boulons, à une embase réalisée à la partie inférieure du chapeau coiffant la cheminée. Selon un autre mode de fixation, une couronne (21) en ce matériau selon l'invention peut être disposée grâce à un collier de serrage (22) contre le chapeau (10) coiffant la cheminée et peut être cousue aux différents secteurs angulaires de sorte que la couronne et la couche de tissu recouvrant le lit catalytique forment un seul morceau.

La partie excédentaire du tissu relevée contre la cheminée matérialisée par cette couronne (21) ne comportant pas de collier (22) peut être recouverte, comme le montre la figure 4, par une autre couronne (21 a) de tissu dont la partie supérieure adhère à la cheminée (9) ou au chapeau (10) grâce à un autre collier (22) et dont le reste recouvre l'extrémité correspondant à la partie excédentaire du tissu. Ainsi, une déformation de la surface du lit catalytique consécutive à un éventuel tassement du catalyseur peut être compensée par le coulissement du tissu appliqué contre le chapeau ou la cheminée sous la couronne.

La couronne de tissu (21a), libre de tout mouvement peut constituer aussi la partie cylindrique d'un chapeau (10) en tissu réfractaire selon l'invention, comme cela est montré dans la figure 5, qui coiffe la cheminée (9), la partie de tissu de la couronne (21) coulissant sous la couronne (21a) du chapeau.

Selon la figure 6, l'extrémité du tissu appliquée contre la cheminée ou le chapeau correspondant à la partie excédentaire par rapport à la surface du lit catalytique est ajustée de façon à avoir une longueur telle qu'elle constitue un soufflet de sécurité (25) permettant de remédier aux éventuels tassements du catalyseur.

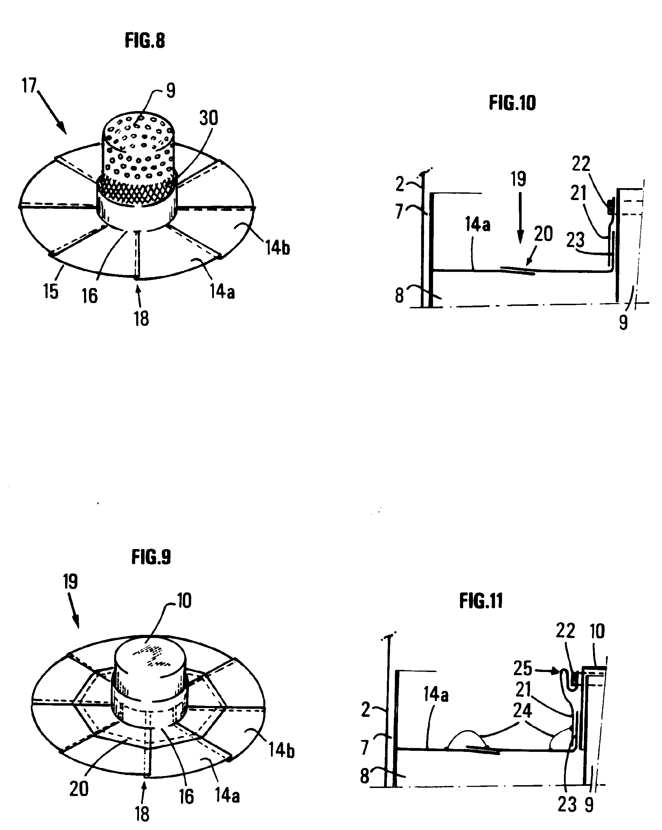

Enfin selon la figure 7, la couverture (19) du lit catalytique, la couronne (21) et le chapeau (10) avec son soufflet de sécurité peuvent former un ensemble d'un seul morceau de tissu convenablement agencé et rendu étanche par des coutures connues de l'Homme de l'Art. Cette solution technique est avantageuse car cet ensemble peut être très facilement installé et retiré du réacteur.

Selon un mode de réalisation particulier, la couche de tissu et la couronne (21a) peuvent être réliées par un fil (24) non destructible par la chaleur pour être facilement remontés avec la cheminée ou son chapeau (figure 4).

On a illustré le cas où la partie excédentaire de la couche de tissu était appliquée contre la cheminée ou le chapeau. Il est bien entendu qu'une partie excédentaire de la bande peut éventuellement être appliquée contre la couronne extérieure de distribution de la charge, de façon à servir de sécurité, comme illustré par la figure 7.

Selon le deuxième mode de mise en oeuvre, les références des figures 1 à 7 sont utilisées pour les figures 8 à 11 lorsqu'elles indiquent les mêmes moyens.

Sur la troisième couche de billes constituée de support de catalyseur inerte que présente le fond intérieur (6 figure 1) du réacteur, sont disposées des bandes du matériau selon l'invention formant une couche souple (17) qui sont des secteurs annulaires (14a, 14b) dont l'extrêmité (15) côté couronne de distribution de la charge est sensiblement en contact avec cette couronne (7) et dont l'extrêmité (16) côté cheminée est redressée vers le haut du réacteur et s'applique sur une hauteur de 8 cm environ sur la cheminée (9).

Les différentes sections annulaires (14a et 14b) sont superposées sur une partie de leur largeur de sorte qu'elles forment la couche (17) avec des zones de superposition (18) selon un cercle dont la largeur de recouvrement a une largeur au moins égale à l'amplitude de la déformation qui peut être imposée de sorte que l'étanchéité axiale est assurée. Le catalyseur repose sur cette couche (17) de tissu et son poids contribue à l'étanchéité axiale de la paroi inférieure du compartiment.

Le fond supérieur 4 du réacteur comprend au dessus du lit catalytique sur lequel elle s'appuie, une couche 19 de l'assemblage souple de tissus et de feutre en sandwich décrit ci-haut sur laquelle repose les couches de billes décrites ci-haut qui contribuent avec les bandes de la couche souple à une étanchéité axiale du lit.

La paroi supérieure du compartiment (figure 9) ou toit du lit (32) comprend donc une couche (19) d'une pluralité de bandes (14a) du matériau souple décrit, de forme globalement annulaire pour épouser la surface du compartiment qu'elles recouvrent. Ces différentes sections de bandes (14a, 14b) sont disposées de façon à déterminer des zones (18) de superposition dont la largeur de recouvrement est au moins égale à l'amplitude de la déformation que le garnissage peut imposer au cours de la réaction ou à la suite d'une manoeuvre intempestive.

La superposition des bandes peut être radiale également et la zone de recouvrement (20) peut avoir une largeur au moins égale à la déformation que le garnissage va imposer. Il s'en suit que la surface totale de la pluralité de bandes ainsi disposées est supérieure par exemple d'environ 50 % à la surface annulaire du compartiment catalytique.

Les bandes côté cheminée peuvent être fixées par des moyens adéquats tels que des boulons, à une embase réalisée à la partie inférieure du chapeau coiffant la cheminée.

Selon la figure 10, la longueur des bandes constituant la couverture selon la direction radiale du compartiment peut excéder l'épaisseur du lit catalytique par exemple de 40 à 50 cm ; par exemple l'extrémité côté cheminée, correspondant à la partie excédentaire (23) est alors redressée vers le haut du réacteur et plaquée contre la cheminée (9). Elle est recouverte d'une couronne (21) constituée de matériau selon l'invention dont la partie inférieure recouvre la partie excédentaire relevée de telle façon que celle-ci coulisse sous la couronne selon le tassement du catalyseur. Cette couronne peut adhérer à la cheminée centrale (9) par sa partie supérieure qui peut être fixée par un collier de serrage (22) ou par une cordelette en fibres de matière céramique. Un chapeau métallique (figures 6, 10) peut coiffer la partie supérieure de la cheminée. Il épouse en général la forme sensiblement cylindrique de la cheminée et est adapté à être sensiblement étanche vis-à-vis du gaz et de l'effluent. Il présente dans ces conditions l'avantage d'être de faible hauteur. Ce chapeau étanche peut aussi être constitué par le matériau souple en matière céramique selon l'invention.

Selon un autre mode de réalisation, semblable à celui de la figure 5, la partie excédentaire (23) des bandes est appliquée contre la cheminée (9) et recouverte par la partie cylindrique ou couronne d'un chapeau (10) en ce matériau en matière céramique sensiblement étanche qui coiffe la cheminée. Ce chapeau présente au moins un bourrelet de façon à s'allonger le cas échéant, comme dans le cas de la figure 6.

Selon une variante illustrée par la figure 11, la longueur des bandes selon la direction radiale du compartiment peut excéder l'épaisseur du lit catalytique, par exemple de 20 à 30 cm. L'extrémité côté cheminée peut alors être rédressée vers le haut du réacteur et plaquée contre le chapeau de la cheminée. Une bande de même matériau de forme sensiblement rectangulaire est appliquée contre la partie redressée (23) de la bande formant ainsi la couronne (21). Sa partie supérieure adhérant au chapeau par un collier de serrage ou par une cordelette en matière céramique non destructible de sorte que la bande constituant le toit du compartiment peut coulisser le long du chapeau au gré du tassement du catalyseur.

Selon une variante du dispositif, illustrée par les figures 8, 9 et 10 une partie d'une bande peut reposer sur une partie d'une bande contiguë de sorte que la couverture du compartiment est assurée par une couche de bandes de tissu pouvant glisser les unes sur les autres selon une direction circulaire (figure 8) et/ou radiale (figures 9 et 10).

Selon une autre variante du dispositif, deux couches de bandes de tissu peuvent être superposées. Une bande de la couche supérieure repose sur une partie de la largeur de deux bandes contiguës de la couche inférieure, ces deux bandes pouvant être jointives ou non. Le plan de couverture réalisé selon ces deux variantes peut être horizontal ou incliné vers l'enveloppe ou la cheminée.

Des bandes, côté enveloppe, peuvent être taillées de manière à épouser la forme des moyens de distribution. On peut ainsi rajouter des éléments venant épouser la forme de ces moyens et se superposant aux bandes de tissu.

De manière générale, la mise en place de la tête du compartiment ou du fond du compartiment peut être réalisée comme il a été indiqué ci-dessus et une couture radiale et éventuellement circulaire des éléments de tissu qui se superposent est effectuée par du fil généralement autodestructible lors de cette mise en place. Les fils de couture reliant les diverses bandes perdront toute résistance après la montée en température du lit catalytique afin de laisser les bandes de tissu glisser les unes sur les autres pour donner de la souplesse à l'ensemble ainsi réalisé. Selon un mode de réalisation particulier, illustré par la figure 11, les différentes bandes ou éléments constitutifs de la couverture du lit ou du fond du lit peuvent être réunis entre eux par un fil de liaison (24) non destructible de longueur en général au moins égale à la dimension des zones superposées, ce qui facilite leur récupération lors du déchargement du réacteur. Ces différentes bandes peuvent également être reliées par tout moyen approprié au chapeau de la cheminée. Ainsi, lorsque celui-ci sera remonté, il pourra entraîner la couverture du lit.

Selon un autre mode de réalisation, la partie de bande (23) appliquée contre la cheminée ou le chapeau de la cheminée peut présenter un soufflet de sécurité (25) assurant une plus grande liberté de mouvement.

Ce soufflet de garde (25) peut aussi être disposé sur la couronne (21) recouvrant la partie excédentaire des bandes redressées contre la cheminée ou le chapeau (figure 11) suivant le mode de réalisation choisi.

On a réprésenté la figure 1 avec des billes ou des particules (11, 12) reposant sur la couche souple (19) en guise de paroi supérieure (32), mais on aurait pu représenter la paroi supérieure sans la présence de billes ou de particules.

De même, on aurait pu représenter la paroi inférieure 33 sans les diverses couches de billes 11, 12, 13, la couche de matériau souple reposant directement sur du ciment remplissant le fond du réacteur jusqu'à un niveau sensiblement horizontal.

## Revendications

1. Réacteur pour effectuer des réactions chimiques en phase gazeuse, à une témpérature donnée comprenant une entrée (3) d'une charge gazeuse et une sortie (9) d'un effluent, une enveloppe (7) annulaire de distribution de la charge en communication avec l'entrée de ladite charge, au moins un compartiment (8) annulaire entre ladite enveloppe et ladite sortie comprenant au moins un garnissage (31) sous forme de particules, dans lequel circule radialement ou transversalement au moins un gaz ou un liquide, le compartiment comportant en outre une paroi supérieure (32), caractérisé en ce qu'il comporte, en guise de paroi supérieure, au moins une couche d'au moins un matériau souple (19) de forme appropriée, en matière réfractaire, sensiblement inerte et sensiblement imperméable ou ayant une texture et une porosité telles que ladite couche crée une perte de charge supérieure à celle engendrée par le garnissage de particules, ladite couche de matériau étant disposée sur le garnissage (31) de telle façon qu'elle assure un recouvrement sensiblement total dudit garnissage.

2. Réacteur selon la revendication 1 caractérisé en ce que ladite couche de matériau coopère avec une première couche sensiblement inerte de billes (11, 12) ou de particules de granulométrie et de poids appro-

EP 0 461 988 B1

priés qui repose sur ledit matérieau (19).

3. Réacteur selon la revendication 1 ou 2, caractérisé en ce que le compartiment comporte en outre une paroi inférieure (33) comprenant au moins une couche (17) dudit matériau souple de forme appropriée qui coopère éventuellement avec une deuxième couche sensiblement inerte de billes (11, 12, 13) ou de particules de granulométrie appropriée et qui repose sur ladite deuxième couche inerte de façon à assurer un recouvrement sensiblement total de la deuxième couche.

4. Réacteur selon l'une des revendications 1 à 3 dans lequel ladite couche est constituée d'un seul morceau de forme appropriée ou d'une pluralité de morceaux cousus de telle façon qu'ils constituent un seul morceau dudit matériau sensiblement étanche.

5. Réacteur selon l'une des revendication 1 à 3, dans lequel la couche de matériau souple comporte une pluralité de bandes (14a, 14b) ou de pièces de tissu disposées de telle façon qu'elles soient au moins en partie superposées de manière à glisser les unes sur les autres dans au moins une direction.

6. Réacteur selon la revendication 5 dans lequel lesdites bandes déterminent des zones de superposition (18) ayant une largeur au moins égale à l'amplitude de la déformation que le garnissage peut imposer.

7. Réacteur selon la revendication 5 ou 6 dans lequel la largeur des zones de superposition (18) est comprise entre 0,001 et 0,20 fois la hauteur du garnissage dans le compartiment ou dans lequel la largeur des zones de superposition est de 0,05 à 0,5 fois la longueur ou la largeur d'une bande selon que le recouvrement est réalisé selon une direction respectivement radiale ou circulaire.

8. Réacteur selon l'une des revendications 5 à 7, dans lequel une partie d'une bande ou d'une pièce de tissu repose sur une partie d'une bande ou d'une pièce contiguë.

9. Réacteur selon l'une des revendications 5 à 7, dans lequel deux couches de bandes de tissu sont superposées et dans lequel une bande de la couche supérieure repose sur une partie de la largeur de deux bandes contiguës de la couche inférieure.

10. Réacteur selon l'une des revendications 5 à 7, dans lequel deux couches de bandes de tissu sont superposées et dans lequel une partie d'une bande et une partie d'une autre bande contiguë de la couche supérieure recouvrent au moins en partie une bande d'une couche inférieure.

11. Réacteur selon l'une des revendications 1 à 10, dans lequel le compartiment a une section de forme sensiblement annulaire avec une enveloppe de distribution du gaz ou du liquide tout autour du compartiment et une cheminée centrale (9) sensiblement cylindrique comportant des moyens de collecte d'un effluent, caractérisé en ce que la dimension des bandes selon le rayon de la section annulaire est supérieure à l'épaisseur du garnissage, l'extrémité de chaque bande côté cheminée étant recouverte par une couronne (21) en tissu en matière réfractaire dont la partie inférieure recouvre ladite extrémité, de telle façon que cette extrémité coulisse sous la couronne ou bien l'extrémité de chaque bande côté cheminée recouvrant la partie inférieure d'une couronne de tissu en matière réfractaire de telle façon que ladite extrémité coulisse sur la couronne.

12. Réacteur selon la revendication 11, dans lequel la couronne a une partie supérieure adhérant à la cheminée centrale.

13. Réacteur selon l'une des revendications 11 à 12 dans lequel la cheminée est recouverte par un chapeau 10 métallique ou par un chapeau constitué dudit matériau sensiblement étanche.

14. Réacteur selon la revendication 11 dans lequel la couronne est constituée par un chapeau en ledit matériau adapté à être sensiblement étanche vis à vis du gaz et de l'effluent.

15. Réacteur selon l'une des revendications 11 à 14 dans lequel l'extrémité correspondant à la partie excédentaire (21) du matériau est relevée et est fixée à la cheminée ou au chapeau coiffant la cheminée.

16. Réacteur selon la revendication 11, dans lequel l'extrémité correspondant à la partie excédentaire du matériau est agencée au moyen de coutures appropriées de façon à constituer un chapeau sensiblement étanche.

9

17. Réacteur selon l'une des revendications 11 à 16 dans lequel l'extrémité correspondant à la partie excédentaire du matériau présente une longueur telle qu'elle constitue un soufflet de sécurité (25).

18. Réacteur selon l'une des revendications 1 à 17, dans lequel le compartiment a une section de forme sensiblement rectangulaire ou carrée.

19. Réacteur selon l'une des revendications 1 à 18 dans lequel ladite couche de matériau souple est constituée d'au moins un tissu.

20. Réacteur selon la revendication 19 dans lequel le tissu est à base de fibres en matière céramique.

21. Réacteur selon l'une des revendications 19 à 20 dans lequel le tissu est rendu sensiblement étanche par dépôt d'une couche d'alumine.

22. Réacteur selon l'une des revendications 1 à 18 dans lequel ladite couche de matériau est constituée d'au moins un matériau de type feutre.

23. Réacteur selon l'une des revendications 1 à 18 dans lequel ladite couche comprend au moins un matériau de type feutre disposé en sandwich entre au moins deux tissus, assemblés par couture.

24. Utilisation du réacteur selon l'une des revendications 1 à 23 dans un procédé de synthèse du méthanol et dans un procédé de réformage d'une coupe pétrolière.


**Patentansprüche**

1. Reaktor zur Durchführung chemischer Reaktionen in gasförmiger Phase bei einer gegebenen Temperatur mit einem Eintritt (3) für eine gasförmige Charge und einem Austritt (9) für einen Abstrom, einem Ringverteilermantel (7) für die Charge in Verbindung mit dem Eintritt dieser Charge, wenigstens einer Ringkammer (8) zwischen diesem Mantel und diesem Austritt, die wenigstens eine Auskleidung (31) in Form von Partikeln umfaßt, in der radial oder quer wenigstens ein Gas oder eine Flüssigkeit zirkulieren, wobei die Kammer im übrigen eine obere Wand (32) umfaßt, dadurch gekennzeichnet, daß er anstatt der oberen Wand wenigstens eine Schicht aus wenigstens einem nachgiebiger. Material (19) geeigneter Form, aus feuerfestem Material, die im wesentlichen inert und im wesentlichen impermeabel ist, umfaßt, oder die eine Textur und eine Porosität der Art hat, daß diese Schicht einen Druckverlust größer als den durch die Auskleidung der Partikel erzeugten hervorruft, wobei die Materialschicht auf der Auskleidung (31) derart angeordnet ist, daß sie eine im wesentlichen vollständige Überdeckung dieser Auskleidung sicherstellt.

2. Reaktor nach Anspruch 1, dadurch gekennzeichnet, daß diese Materialschicht mit einer ersten, im wesentlichen inerten Schicht von Kugeln (11,12) oder Partikeln geeigneter Granolometrie und geeigneten Gewichts, die auf diesem Material (19) ruht, zusammenwirkt.

3. Reaktor nach Anspruch 1 oder 2, dadurch gekennzeichnet, daß die Kammer im übrigen eine untere Wandung (33) umfaßt, die wenigstens eine Schicht (17) dieses nachgiebigen Materials geeigneter Form umfaßt, die gegebenenfalls mit einer zweiten, im wesentlichen inerten Schicht von Kugeln (11,12,13) oder Partikeln geeigneter Granolometrie zusammenwirkt und die auf dieser zweiten inerten Schicht derart ruht, daß eine im wesentlichen vollständige überdeckung der zweiten Schicht sichergestellt ist.

4. Reaktor nach einem der Ansprüche 1 bis 3, bei dem diese Schicht gebildet wird durch ein einziges Stück geeigneter Gestalt oder eine Vielzahl von zusammengenähten Stücken, derart, daß sie ein einziges Stück dieses im wesentlichen dichten Materials bilden.

5. Reaktor nach einem der Ansprüche 1 bis 3, bei dem die Schicht aus nachgiebigem Material eine Vielzahl von Bändern (14a, 14b) oder gewebten Teilen derartig umfaßt, daß sie wenigstens zum Teil derart überlagert sind, daß sie aufeinander in wenigstens einer Richtung gleiten.

6. Reaktor nach Anspruch 5, bei dem diese Bänder Überlagerungszonen (18) mit einer Breite wenigstens gleich der Verformungsamplitude, welche die Auskleidung veranlassen kann, bestimmen.

7. Reaktor nach Anspruch 5 oder 6, bei dem die Breite der Überlagerungszonen (18) zwischen dem 0,001- und 0,20-fachen der Höhe der Auskleidung in der Kammer beträgt oder bei dem die Breite der überlagerungszonen zwischen dem 0,05- bis 0,5-fachen der Länge oder der Breite eines Bandes ausmacht, je nachdem, ob die Überdeckung gemäß einer radialen oder kreisförmigen Richtung realisiert ist.

8. Reaktor nach einem der Ansprüche 5 bis 7, bei dem ein Teil eines Bandes oder gewebten Teils auf einem benachbarten Band oder benachbarten Teil aufruht.

9. Reaktor nach einem der Ansprüche 5 bis 7, bei dem die Gewebebandschichten überlagert sind und bei dem ein Band der oberen Schicht auf einem Teil der Breite der beiden benachbarten Bänder der unteren Schicht ruht.

10. Reaktor nach einem der Ansprüche 5 bis 7, bei dem zwei Gewebebandschichten überlagert sind und bei dem ein Teil eines Bandes und ein Teil eines anderen benachbarten Bandes der oberen Schicht wenigstens zum Teil ein Band einer unteren Schicht überdecken.

11. Reaktor nach einem der Ansprüche 1 bis 10, bei dem die Kammer einen Querschnitt im wesentlichen ringförmiger Gestalt mit einem Verteilermantel für Gas oder Flüssigkeit ganz um die Kammer herum und einen zentralen, im wesentlichen zylindrischen Kamin (9) mit Sammlermitteln für einen Abstrom hat, dadurch gekennzeichnet, daß die Abmessung der Bänder gemäß dem Radius des Ringquerschnittes größer als die Dicke der Auskleidung ist, wobei das Ende jedes Bandes kaminseitig durch eine Kranzausbildung (21) aus Gewebe aus feuerfestem Material abgedeckt ist, dessen unterer Teil dieses Ende derart überdeckt, daß dieses Ende unter der Kranzausbildung gleitet oder daß das Ende jedes kaminseitig den unteren Teil einer Gewebekranzausbildung aus feuerfestem Material überdeckenden Bandes derart ist, daß dieses Ende auf der Kranzausbildung gleitet.

12. Reaktor nach Anspruch 11, bei dem die Kranzausbildung einen oberen, am mittigen Kamin haftenden Teil hat.

13. Reaktor nach einem der Ansprüche 11 bis 12, bei dem der Kamin durch einen metallischen Hut (10), oder einen Hut abgedeckt ist, der aus diesem im wesentlichen dichten Material gebildet ist.

14. Reaktor nach Anspruch 11, bei dem die Kranzausbildung gebildet wird durch einen Hut aus diesem Material einer Auslegung, derart, daß es im wesentlichen dicht gegenüber Gas und Abstrom ist.

15. Reaktor nach einem der Ansprüche 11 bis 14, bei dem das dem überschüssigen Teil (21) des Materials entsprechende Ende angehoben und am Kamin oder an dem den Kamin abdeckenden Hut befestigt ist.

16. Reaktor nach Anspruch 11, bei dem das Ende entsprechend dem überschüssigen Teil des Materials derart mit geeigneten Nähten ausgestattet ist, daß ein im wesentlichen dichter Hut gebildet wird.

17. Reaktor nach einem der Ansprüche 11 bis 16, bei dem das dem überschüssigen Teil des Materials entsprechende Ende eine Länge derart aufweist, daß es einen Sicherheitsbalgen (12) bildet.

18. Reaktor nach einem der Ansprüche 1 bis 17, bei dem die Kammer einen im wesentlichen rechteckigen oder quadratischen Querschnitt hat.

19. Reaktor nach einem der Ansprüche 1 bis 18, bei dem diese nachgiebige Materialschicht gebildet wird durch wenigstens ein Gewebe.

20. Reaktor nach Anspruch 19, bei dem das Gewebe auf der Basis von Fasern aus Keramikmaterial ist.

21. Reaktor nach einem der Ansprüche 19 bis 20, bei dem das Gewebe im wesentlichen dicht durch Abscheiden einer Aluminium-oxydschicht gemacht ist.

22. Reaktor nach einem der Ansprüche 1 bis 18, bei dem diese Materialschicht gebildet wird durch wenigstens ein Material vom Typ Filz.

23. Reaktor nach einem der Ansprüche 1 bis 18, bei dem diese Schicht wenigstens ein Material vom Typ Filz umfaßt, das sandwichartig zwischen wenigstens zwei durch Nähen zusammengefügten Geweben angeordnet ist.

24. Verwendung des Reaktors nach einem der Ansprüche 1 bis 23 in einem Verfahren zur Synthese von Methanol und einem Verfahren zum Reformieren einer Erdölfraktion.

## Claims

1. Reactor for performing gas phase chemical reactions at a given temperature, comprising an input (3) of a gaseous charge and an output (9) of an effluent, an annular casing (7) for distributing the charge which communicates with the input of said charge, at least one annular compartment (8) between said casing and said output with at least one lining (31) in the form of particles, in which radially or transversely circulates at least one gas or liquid, the compartment also having an upper wall (32), characterized in that it has as the upper wall at least one layer of at least one appropriately shaped flexible refractory material (19), which is substantially inert and substantially impermeable or which has a texture and a porosity such that the said layer produces a pressure drop higher than that produced by the particulate lining, said material layer being placed on the lining (31) so as to ensure a substantially total covering of the said lining.

2. Reactor according to claim 1, characterized in that said material layer cooperates with a first substantially inert layer of balls (11, 12) or particles having an appropriate grain size and weight and which rests on the said material (19).

3. Reactor according to claims 1 or 2, characterized in that the compartment also has a lower wall (33) with at least one layer (7) of said appropriately shaped flexible material, which optionally cooperates with a second substantially inert layer of balls (11, 12, 13) or particles having an appropriate grain size and which rests on said second inert layer so as to ensure a substantially total covering of the second layer.

4. Reactor according to any one of the claims 1 to 3 wherein the said layer is constituted by a single appropriately shaped piece or a plurality of pieces sewn so as to form a single piece of the said substantiallytight material.

5. Reactor according to any one of the claims 1 to 3, wherein the flexible material layer has a plurality of strips (14a, 14b) or fabric pieces disposed in such a way that they are at least partly superimposed, so as to slide on one another in at least one direction.

6. Reactor according to claim 5, wherein the said strips define superimposing zones (18) having a width at least equal to the extent of the deformation which can be imposed by the lining.

7. Reactor according to claims 5 or 6, wherein the width of the superimposing zones (18) is between 0.001 and 0.20 times the height of the lining in the compartment or in which the width of the superimposing zones is 0.05 to 0.5 times the length or width of a strip, as a function of whether the said covering takes place in a respectively radial or circular direction.

8. Reactor according to any one of the claims 5 to 7, wherein part of a strip or a fabric piece rests on a portion of a strip or a contiguous piece.

9. Reactor according to any one of the claims 5 to 7, wherein two layers of fabric strips are superimposed and wherein one strip of the upper layer rests on a portion of the width of two oontiguous strips of the lower layer.

10. Reactor according to any one of the claims 5 to 7, wherein two layers of fabric strips are superimposed and wherein a portion of a strip and a portion of another contiguous strip of the upper layer at least partly cover a strip of a lower layer.

11. Reactor according to any one of the claims 1 to 10, wherein the compartment has a substantially annular cross-section with a liquid or gas distribution envelope all around the compartment and a substantially cylindrical central stack (9) having effluent collecting means, characterized in that the size of the strips in accordance with the radius of the annular cross-section is greater than the thickness of the lining, the end of each strip on the stack side being covered by a refractory fabric ring (21), whereof the lower portion covers the said end, so that said end slides beneath the ring, or the end of each strip on the stack side covers the lower portion of a refractory material fabric ring in such a way that said end slides on the ring.

**12.** Reactor according to claim 11, wherein the ring has an upper portion adhering to the central stack.

**13.** Reactor aocording to either of the claims 11 and 12, wherein the stack is covered by a metal cap (10) or by a cap made from the said substantially tight material.

**14.** Reactor according to claim 11, wherein the ring is constituted by a cap made from the material which can be substantially tight with respect to the gas and the effluent.

**15.** Reactor according to any one of the claims 11 to 14, wherein the end corresponding to the excess part (21) of the material is raised and fixed to the stack or to the cap covering the stack.

**16.** Reactor according to claim 11, wherein the end corresponding to the excess portion of the material is arranged by means of appropriate seams so as to constitute a substantially tight cap.

**17.** Reactor according to any one of the claims 11 to 16, wherein the end corresponding to the excess portion of the material has a length such that it constitutes a safety blower (25).

**18.** Reactor according to any one of the claims 1 to 17, wherein the compartment has a substantially rectangular or square cross-section.

**19.** Reactor according to any one of the claims 1 to 18, wherein the said flexible material layer is constituted by at least one fabric.

**20.** Reactor according to claim 19, wherein the fabric is based on ceramic material fibres.

**21.** Reactor according to either of the claims 19 and 20, wherein the fabric is made substantially tight by the deposition of an alumina layer.

**22.** Reactor according to any one of the claims 1 to 18, wherein the said material layer is constituted by at least one felt-type material.

**23.** Reactor according to any one of the claims 1 to 18, wherein the layer comprises at least one felt-type material arranged in sandwich form between at least two fabrics assembled by sewing.

**24.** Use of the reactor according to any one of the claims 1 to 23 in a process for the synthesis of methanol and in a process for reforming a petroleum fraction.

**FIG.1**

**FIG.4**

**FIG.5**

**FIG.2**

**FIG.7**

**FIG.3**

**FIG.6**

**FIG.8**

**FIG.10**

**FIG.9**

**FIG.11**